# EUROPEAN PATENT APPLICATION

(11) **EP 2 518 140 A1**
(43) Date of publication of application: **31.10.2012**
(21) Application number: 12172874.5
(22) Date of filing: 09.11.2007
(51) Int. Cl.: C12N 5/077

(54) **Dedifferentation of adult mammalian cardiomyocytes into cardiac stem cells**

(30) Priority: 09.11.2006 US 858006 P
(62) Divisional of application: 07871426.8
(71) Applicant: The Johns Hopkins University, Baltimore, MD 21201 (US)
(72) Inventor: Marban, Eduardo, Beverly Hills, CA 90210 (US)
(74) Representative: Tombling, Adrian George

(57) **Abstract**

Dedifferentiation is a mechanism whereby specialized cells regain properties of their ancestors, including, in the extreme, stemness. We found that highly-purified cardiomyocytes isolated from adult mammalian hearts dedifferentiated rapidly when cultured in mitogen-rich medium. Such myocytes reentered the cell cycle and proliferated, expressing stem cell surface markers such as c-kit and early cardiac transcription factors including GATA and NKx2.5. These myocyte-derived cells (MDC) were capable of re-differentiating into myocytes and endothelial cells. Contrary to prevailing dogma, cardiomyocyte dedifferentiation yields proliferative cells expressing stem cell markers and capable of multilineage differentiation. Cardiomyocyte dedifferentiation is a potential source of endogenous stem cells in the adult heart.

## Description

This invention was made using funds from the United States government, which therefore retains certain rights in the invention. A grant from the National Institutes of Health, HL083109, was used.

### TECHNICAL FIELD OF THE INVENTION

This invention is related to the area of stem cells and stem-like cells. In particular, it relates to cardiac cells having regenerative uses.

### BACKGROUND OF THE INVENTION

The mammalian heart has long been considered to be a highly specialized organ unable to repair itself after injury. The recent recognition that the heart contains its own pool of stem cells has ushered in a new era of cardiac biology and therapeutics. Cardiac stem cells (CSCs) express a variety of stem cell antigens (e.g. c-kit, sca-1, isl-1, SSEA-1, ABCG2) and cardiac-specific markers (e.g. NKx2.5, GATA4, α-MHC) (Lyngbaek et al., 2007; Barile et al., 2007); when transplanted, they contribute to regeneration of injured myocardium and improve cardiac function. Nevertheless, little is known regarding the sources of cardiac stem cells. The focus to date has been on seeding from circulating blood pools (Yeh et al., 2003; Shyu et al., 2006) versus endogenous cardiac origin, e.g. as embryonic remnants (Torella et al., 2006).

Here, we consider dedifferentiation as yet another potential source of CSCs. Dedifferentiation can change the phenotype and functions of specialized cells, rendering them closer to their ancestors with augmented plasticity. For instance, pigment cells derived from neural crest can dedifferentiate and reprogram to become multipotent self-renewing progenitors expressing early neural marker genes Sox10, FoxD3, Pax3 and Slug, and give rise to glial cells and myofibroblasts (Real et al., 2006). Dedifferentiation is a common occurrence in plants; plant protoplasts from tobacco leaves have been reported to undergo a transitory phase conferring pluripotentiality, that precedes signal-dependent re-entry into the cell cycle (Zhao et al., 2001).

In adult cardiomyocytes, dedifferentiation has been investigated extensively at the phenotypic level. Compared to normal myocytes, dedifferentiated cells become physiologically more "neonatal", while morphologically they flatten and spread in culture, with increased diameter and surface area (Ausma et al., 2001; Fredj et al., 2005). Sarcomeric structures are lost, with disorganized myofibrils (Bird et al., 2003; Horackova and Byczko, 1997) and dramatically altered expression of cardiac α-actinin, α-MHC, α-MLC, etc (Benardeau et al., 1997b; Bird et al., 2003). A phenomenon akin to *in vitro* dedifferentiation has also been described *in vivo,* in fibrillating atria (Rucker-Martin et al., 2002), chronically-ischemic myocardium, and in the border zone of myocardial infarcts (Dispersyn et al., 2002; Driesen et al., 2007). Such dedifferentiated myocytes are not apoptotic and presumably reflect adaptations to abnormal myocardial stress (Dispersyn et al., 2002).

There is a continuing need in the art for new sources of regenerative cells for therapy of heart diseases.

### SUMMARY OF THE INVENTION

According to one embodiment of the invention a method for obtaining stem-cell-like myocyte-derived cells (MDCs) from atrial or ventricular heart tissue is provided. Cells are isolated from atrial or ventricular heart tissue to form a cell suspension. The cell suspension may be optionally purified to increase the proportion of myocytes in the cell suspension. The cells are cultured in a medium comprising a mitogen. A composition comprising MDCs is thereby formed.

According to some embodiments, cells are harvested at a plurality of time points from the medium comprising MDCs to form a plurality of samples of MDCs. The proliferative capacity of one or more of the samples of MDCs is assessed. One or more of the samples of MDCs is then clonally proliferated. One or more of the samples of MDCs is tested to confirm expression of one or more marker of stem cells selected from the group consisting of c-kit, sca-1, MCR1, CD34, CD33, alpha-MHC, NKx2.5, GATA4, and CD105.

Also provided by the present invention is an isolated preparation of cardiac stem-like cells. The cells proliferate in culture and express a marker selected from the group consisting of c-kit, NKx2.5, and GATA4. The cells can be derived from adult cardiac atrial or ventricular myocytes.

These and other embodiments which will be apparent to those of skill in the art upon reading the specification provide the art with methods and tools for regenerating cardiac tissue after disease has damaged cardiac tissue.

### BRIEF DESCRIPTION OF THE DRAWINGS

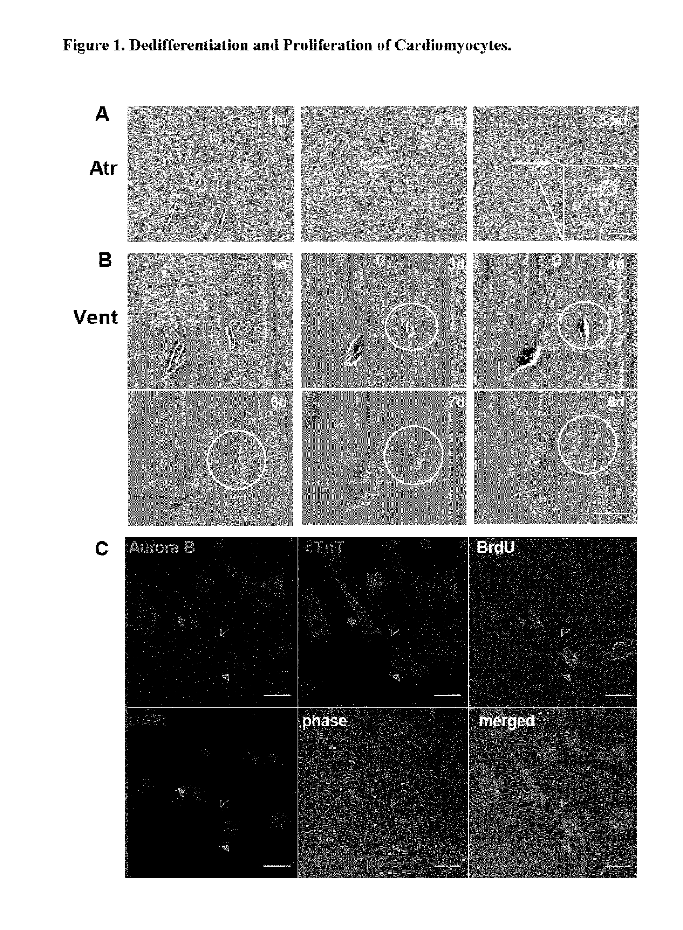

**Fig. 1A-1C****. Dedifferentiation and Proliferation of Cardiomyocytes. (****Fig. 1A****)** Purified atrial myocytes were cultured as described in Experimental Procedures. Daughter cell budded from the mother atrial myocyte after 3.5 days; Arrow indicates the daughter cell. (Fig. 1B) Purified ventricular myocytes (*insert*) dedifferentiate remarkably after about 3 days of culture, and start to divide at day 6, showing significant cytoplasmic division. Scale bar, 100 µm. (**Fig. 1C**) Examples of proliferation of atrial myocytes culture for 6d. Immunofluorescence shows the expression of Aurora B (green) at the cleavage gap (white arrow) between the myocyte that expresses weak cTnT (red; red arrow) and the newly divided cell without detectable cTnT (large white arrow); Both cells are positive to anti-BrdU immunostaining (white). Nuclei are stained with DAPI (blue). Scale bar, 20 µm.

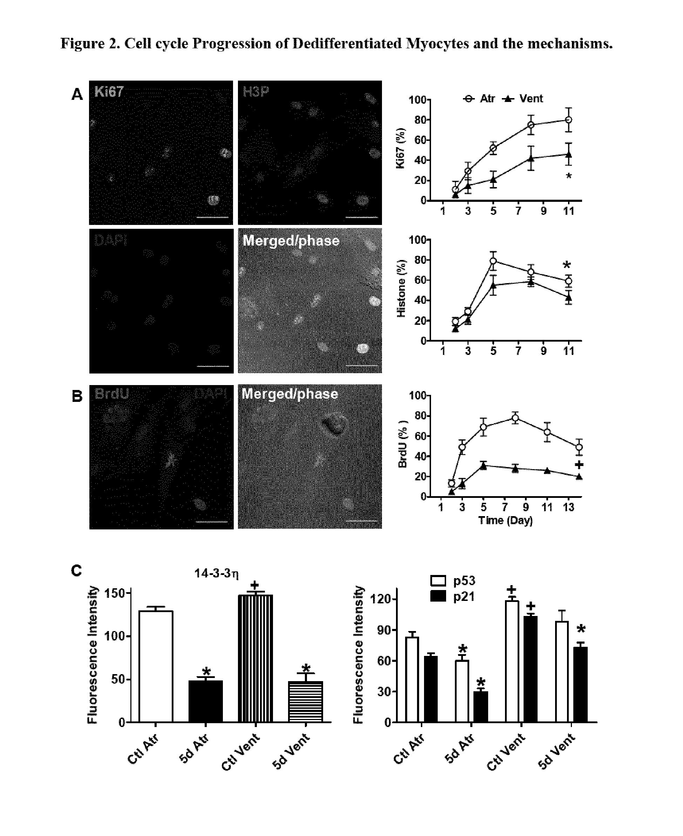

**Fig. 2A-2C****. Cell cycle Progression of Dedifferentiated Myocytes and the mechanisms. (****Fig. 2A, Fig. 2B****)** Expression of cell cycle markers with antibodies against Ki67 (**Fig. 2A**, green) and histone S3 pospho S10 (H3P) (**Fig. 2A**, His, red), and BrdU **(****Fig. 2B**, red). Shown are the representative images of culture of ventricular myocytes at 8d. Plotted data (right panels) show the time course of expression of Ki67, H3P, and BrdU incorporation as percentages of cells. * p<0.05; + p<0.001 for atrial *vs* ventricular myocytes; n=151~380 cells for different time points. **(****Fig. 2C****)** Mean data of fluorescence intensity for the expressions of 14-3-3 (left), and p21 and p53 (right) in freshly isolated (Ctl) atrial myocytes (Atr), which were significantly lower than in ventricular myocytes (Vent); Both decreased significantly after 5d culture. * p<0.01 *vs* Ctl; +p<0.01 *vs* Ctl Atr.

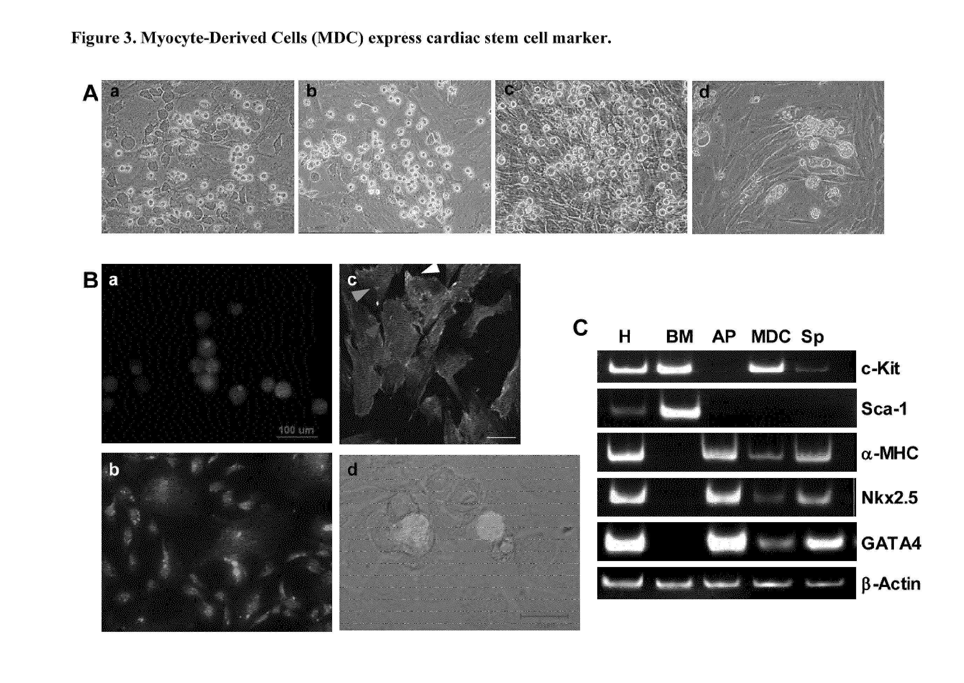

**Fig 3A-3C****. Myocyte-Derived Cells (MDC) express cardiac stem cell marker. (**Fig. 3A) Example images show the clusters of small phase bright cells (MDC) arise from myocytes isolated from guinea pig atria (*a*, 10d culture; b, 4d after MDC 1^{st} harvest), rat atria (c, 9d culture) and ventricle (*d*, 14d culture) in continuous culture. (**Fig 3B**) Expression of c-kit in freshly harvested MDC (a) or plated for 18hr (b); (c) Image shows the heterogenous MDC, expressing c-kit (green), CD34 (white) and cTnT (red); (d) After harvest of MDC, culture layer cells were incubated with c-kit-PE (red), indicating strong c-kit staining in cells located proximal around the MDC clusters being harvested. (**Fig 3C**) RT-PCR amplification of stem cell and cardiac markers. H, heart tissue; BM, bone marrow cells; A.P., purified atrial myocytes; MDC, myocyte-derived cells; Sp, spheres formed from MDC.

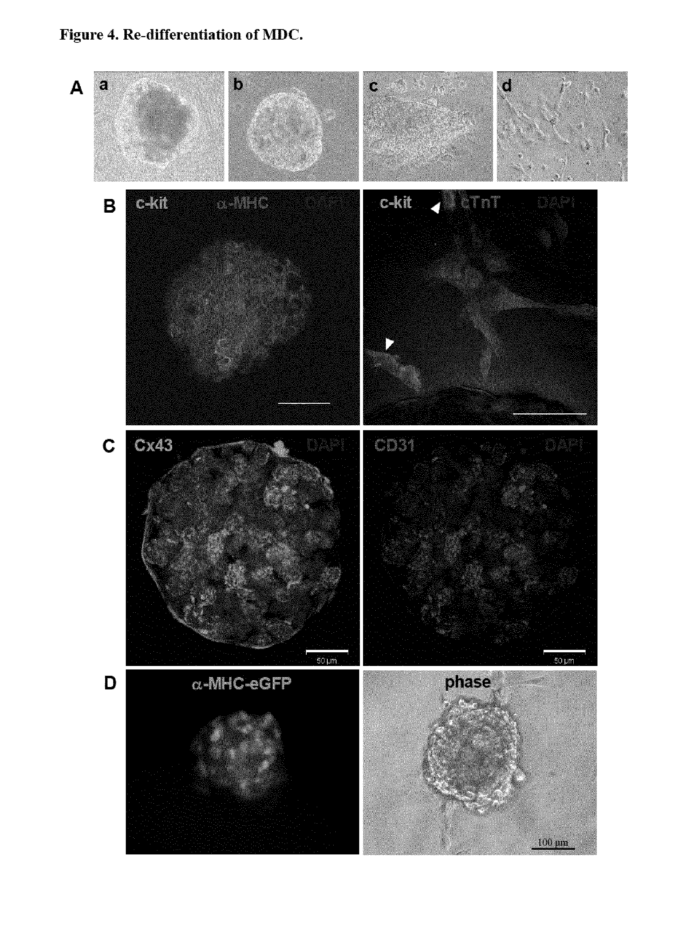

**Fig. 4A-D****. Re-differentiation of MDC. (****Fig. 4A****)** Sphere formed from MDC loosely adhere on the culture layer (*a*) or detached and became suspension, and eventually (2-5d) beat spontaneously. Both MDC and spheres can be harvested and cultured for further tests. *(b)* Freshly harvested MDC sphere; (*c*) MDC sphere flattened on the culture vessel and cells crawled off the sphere 3 hr after plating. (*d*) MDC 18 hr after harvest from myocyte culture. Shown in here are rat myocyte culture. (**Fig. 4B**) Example image of immunohistochemical test showing the expression of c-kit and cardiac α-MHC in sphere (left) and cells off the sphere (right). (**Fig. 4C**) Expression of Cx43 (left) and CD31 (right) in spheres. (**Fig. 4D**) Green fluorescence in a sphere transduced with replication-defective lentivirus encoding eGFP driven by cardiac α-MHC promoter at 3d.

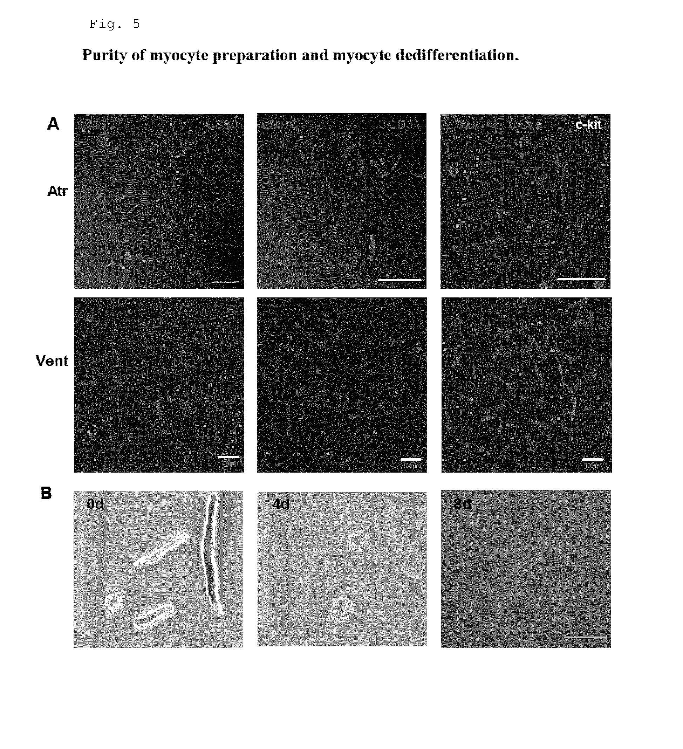

**Fig. 5A-5B** **(S1). Purity of myocyte preparation and myocyte dedifferentiation. (****Fig. 5A****)** Immunocytochemical tests for cardiac α-MHC, CD90, CD34, CD31 or CD90 (all color-coded) in purified atrial (Atr) or ventricular (Vent) myocytes, showing the preparation is highly pure for cardiomyocytes; **(Fig. 5**B) Time-lapse tracking of guinea pig myocyte dedifferentiation, showing significant weaker expression of cTnT.

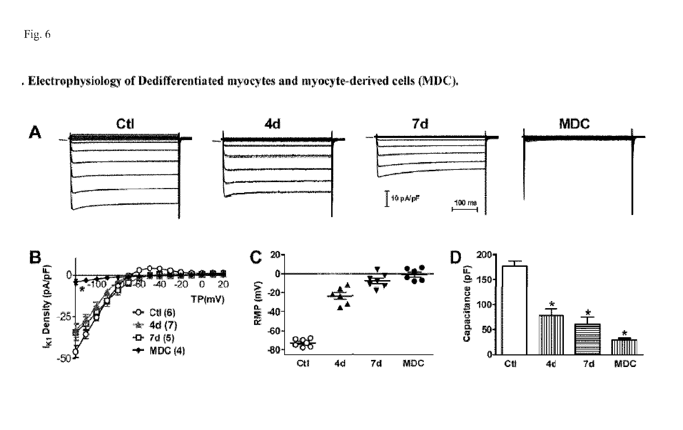

**Fig. 6A-6D** **(S2). Electrophysiology of Dedifferentiated myocytes and myocyte-derived cells (MDC). (****Fig. 6A****)** Example recording of inward rectifier potassium current (I_{K1}) in fresh (Ctl) and 4d or 7d cultured myocytes, and MDC; **(****Fig. 6B**) I-V relationship of I_{K1} in fresh or cultured myocytes or in MDC. Digits in bracket denote cell numbers. * p<0.05. **(****Fig. 6C****)** Resting membrane potential (RMP); p<0.001 for all *vs* Ctl. (**Fig. 6D**) Capacitance as a means to measure cell size

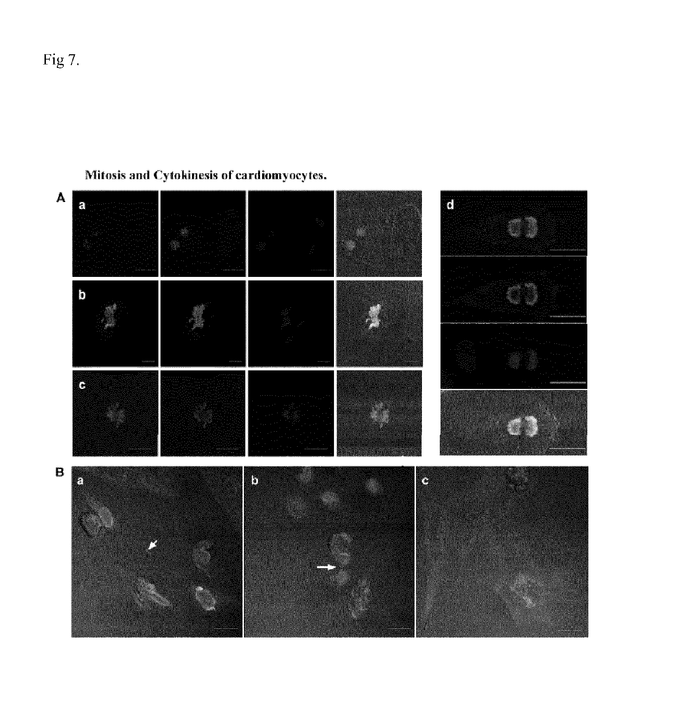

**Fig. 7A(a-d)-7B(a-c)****. (S3) Mitosis and Cytokinesis of cardiomyocytes.**

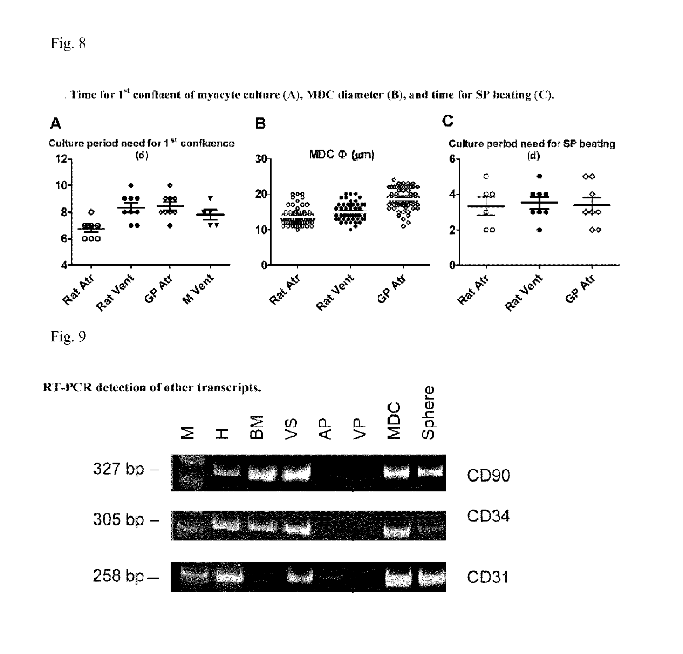

**(****Fig. 8A-8C****)** (S4). Time for 1^{st} confluent of myocyte culture (**Fig**. 8A), MDC diameter (**Fig**. 8B), and time for SP beating (**Fig. 8C**).

**Fig. 9 (S5). RT-PCR detection of other transcripts.** RT-PCR amplification of other markers of rat cells. M, DNA ladder; H, heart; BM, bone marrow; VS, aorta vessel; AP, purified atrial myocytes; VP, purified ventricular myocytes; MDC, myocyte-derived cells; Sphere, sphere formed from MDC.

### DETAILED DESCRIPTION OF THE INVENTION

We investigated dedifferentiation of adult atrial and ventricular myocytes. The salient results are that in vitro cell culture conditions can promote dedifferentiation that is associated with down-regulation of cell cycle inhibitors 14-3-3η and p21, and that the dedifferentiated cells can divide and generate cardiac precursor cells that are positive for c-kit, Nkx2.5 and GATA4. The dedifferentiated adult mammalian cardiomycytes are an abundant source of cells for use in cardiac cell regenerative therapies.

Surprisingly, applicants have found that adult myocytes, derived from the atrium or ventricles, can dedifferentiate and become stem-cell like (MDCs). The stem-cell likeness is reflected in the expression of c-Kit (detectable by RT-PCR), which adult myocytes do not express. When these MDCs differentiate, they lose expression of c― Kit. We have not detected expression of Sca-1 under current conditions in the MDCs, although conditions may be found in which it would be expressed. One distinguishing feature of the MDCs is their cell size. The MDCs (10-30 um) are bigger than regular cardiac stem cells (approx 6-10 um diameter) or bone marrow stem cells (6-8 um).

Myocytes can be isolated from either atrial or ventricles of the heart. These can be obtained from any source, for example from biopsies (endomyocardial or surgical specimens), cadavers, animal donors, etc. As is known in the art, the tissue can be mechanically macerated to produce and liberate myocytes. Enzymes, such as proteases, can also be used to liberate myoctyes from the tissue. Purification of adult myocytes can be by any means known in the art. These include differential centrifugation, culturing under selective conditions, differential harvesting of cultured cells, and gradient centrifugation. The purification, however, is optional.

In order to dedifferentiate isolated adult cardiac myoctyes, one can culture them in the presence of mitogens. Proliferating cells results which have altered properties. Any mitogen can be used. Mitogens present in serum can be used, including bovine, fetal bovine, human, porcine, and ovine sera. Any amount between 0.1 to 20 % serum can be used, for example, from 0.1 to 1 %, from 1 % to 5 %, from 5 % to 10 %, from 10 % to 15 %, and from 15 % to 20 %. The amount can be increased, in steps increases or in a gradient, as growth progresses. Purified growth factors can be used as mitogens, including but not limited to VEGF, HGV, IGF, FGF, EGF, GCSF, GMCSF, MCSF, CSF-1, and PDGF. Changes in proliferation markers, proliferative index, and marker expression can be seen in as little as 3, 5, 7, 9, 11 days. Culturing can be carried out from 1 to 60 days. Cultures can be reseeded to maintain a high proliferative index. Cell cycle inhibitor expression decreases and proliferative index increases from the initial.

The electrophysiology of the cells also changes as they are cultured. Inward rectifier potassium current and membrane resting potential decreased as cells dedifferentiated. In addition, electrical capacitance of the cells decreased.

Cardiomyocytes can be isolated from any mammals. These include rodents and primates. Exemplary animal sources include rat, mouse, guinea pig, goat, rabbit, pig, and human. Cardiomyocytes can be obtained from laboratory animals, cadavers, or patients. If human cardiomyocytes are used, they can be delivered back to the same patient or to different patients. They can be stored at any stage in the process, before dedifferentiation, after dedifferentiation, and after redifferentiation.

The MDCs demonstrate the ability to differentiate. For example, they form spheres. The spheres express less CD34 and c-Kit than the MDCs.

Because the MDCs have the ability to redifferentiate, they are useful for treating patients and animals with heart disease or heart disease models. Such diseases include chronic heart failure, post-myocardial infarction, right ventricular failure, pulmonary hypertension, ventricular dysfunction induced by a cytotoxic agent, and ventricular dysfunction induced by an anti-neoplastic agent. The MDCs can be introduced by any means known in the art, including but not limited to intracoronary infusion via a catheter, intramyocardial injection via a catheter, and intramyocardial injection during surgery.

The above disclosure generally describes the present invention. All references disclosed herein are expressly incorporated by reference. A more complete understanding can be obtained by reference to the following specific examples which are provided herein for purposes of illustration only, and are not intended to limit the scope of the invention.

### EXAMPLES

### EXAMPLE 1-- Dedifferentiated cardiomyocytes re-enter cell cycle and proliferate

We purified enzymatically-separated cardiomyocytes from hearts of adult rats, guinea pigs or mice using multiple differential centrifugation and Percoll gradient separation steps. Tests of morphology (Fig 1), immunoreactivity (Fig S1A), and RT-PCR (Fig 3, Fig S5) confirmed the purity of the isolated cardiomyocytes. Visually, the primary cells look homogeneously large and striated despite that atrial myocytes have variable shapes when plated to culture; more importantly, there is no detectable expression of proteins or transcripts characteristic of fibroblasts, endothelial cells or stem cells. To track individual cells in culture, atrial and ventricular myocytes were cultured at low density in grid-culture dishes or on coverslips. Shortly after plating, myocytes dedifferentiated, losing striations, rounding up and, often, beating spontaneously. Immunocytochemical studies demonstrated that after 3 days of culture, myocytes dedifferentiated, with significantly reduced expression of α-MHC or cTnT (Fig S1B). Inward rectifier potassium current (I_{K1}) and membrane resting potential, characters of cardiomyocytes, were dramatically reduced in dedifferentiated myocytes. Electrical capacitance as a means of assessing cell size (Zhang et al., 2003) was also significant smaller with culture prolonged and dedifferentiation and proliferation progressed (Fig S2).

In addition to these long-recognized morphological and physiological changes, we found that plated myocytes begin to divide and give rise to daughter cells within 3-7 days in culture. Expression of aurora B in the cleavage gap between cells indicates that new divided, BrdU-positive cells with barely detected cTnT are from cardiomyocytes which typically express cTnT (Figure 1). In addition, atrial myocytes showed greater plasticity and produced daughter cells earlier than ventricular myocytes, but the phenomena are generally similar in myocytes from either chamber. A subgroup of dedifferentiated round myocytes that budded off new daughter cells continued to demonstrate spontaneous contractions. In other cases, cells rounded up before flattening and spreading, did not show spontaneous beating, but gave rise to phase-bright daughter cells.

Although the dedifferentiation mechanism has been studied intensively and better elucidated in myocytes from amphibians and zebrafishes (Straube and Tanaka, 2006b; Lien et al., 2006; Ahuja et al., 2007), it is poorly understood in mammalian cardiomyocytes (Engel et al., 2005; Driesen et al., 2006; Montessuit et al., 2004). We analyzed cell cycle progression in this cell culture model by studying the active cell cycle markers Ki67, histone H3 and BrdU incorporation by immunocytochemistry. Ki-67 is a vital molecule for cell proliferation that is expressed in proliferating cells at all phases of the active cell cycle, but is absent in resting (G0 phase) cells. After 2d in culture, 11 ± 8 % and 6 ± 2 % of atrial and ventricular myocytes, respectively, reentered active cell cycle and expressed Ki-67, with gradually increased levels, reaching to 80 ± 11.9 % and 46 ± 11 % at 11 d for atrial and ventricular myocytes, respectively (p<0.001) (Figure 2A). We assessed the proportion of dedifferentiated myocytes entering the S phase by incubating the cells with BrdU for various periods. Cells in M phase were detected using an antibody against phospho histone H3 at S10 (H3P). We found a progressive increase in the numbers of BrdU― and H3P― positive cells, reaching a maximum at about 1 week. Interestingly, the proportion of BrdU- and H3P- positive cells was always higher in cultures of atrial myocytes than in that of ventricular myocytes (Figure 2A, 2B). Besides the cytokinesis, we also found cells in anaphase and telophase (Figure S3), demonstrating the progression of proliferation of the dedifferentiated myocytes.

To further decipher the mechanisms underlying the cell cycle progression and their differences between atrial and ventricular myocytes, we investigated the expressions of interrelated factors like 14-3-3 (YWHAH), p21 and p53 that are critical checkpoint regulators in cell cycle progression (Ahuja et al., 2007) by immunocytochemical detection of cells cultured for 5 days. Expression of the negative cell cycle regulator 14-3-3 has been shown to prevent the cell cycle progression and serum-induced proliferation (Du et al., 2005; Yang et al., 2006). As predicted, the expression of 14-3-3η, an abundant isoforms in the heart (He et al., 2006), was significantly lower in freshly isolated atrial myocytes than in fresh ventricular myocytes. Furthermore, on day 5, when was the faster response period of cell cycle progression for both types of cells, expression of 14-3-3η was dramatically reduced (Fig 2C). p21 (WAF1/CIP1), a downstream target of 14-3-3 and key inhibitory factor involving in all phases of cell cycle (Li and Brooks, 1999), was also reduced significantly in cultured dedifferentiating/ proliferating myocytes. Its endogenous level was 61 % higher in freshly isolated ventricular myocytes than in atrial myocytes. Furthermore, p53 expressed much less in fresh atrial myocytes than in ventricular myocytes, and decreased significantly in atrial myocytes but not much in ventricular myocytes. Taken together, the data suggest the weaker inhibitory signals in atrial myocytes facilitate their faster and easier cell cycle progression and the diminution of the inhibitory factors render the cell into cell cycle progression and proliferation.

### EXAMPLE 2-- Myocyte-derived cells exhibit cardiac stem cell markers

Myocytes cultured in normal density become confluent after 1-2 weeks (Figure S4A) and thereafter clusters of loosely-adherent phase-bright round cells emerged above the monolayer of dedifferentiated/proliferating cells (Figure 3). These cells, seemed to be heterogenous in size (Fig S4B), can be harvested by gently pipetting without trypsinization and are referred to as myocyte-derived cells (MDC).

Dedifferentiation, *e*.*g*., in pigment cells, has been demonstrated to contribute to stem cells and tissue regeneration (Real et al., 2006). We asked if MDC that is distinct from cardiomyocytes in morphology and electrophysiology, have any characteristics of cardiac stem cells (Smith et al., 2007; Boyle et al., 2006). By direct and indirect fluorescent immunostaining, we found that rat MDC do indeed express stem cell markers c-kit and CD34, but little or weak, if any, sca-1 or CD90 (data not shown); 61 ± 19.7% freshly harvested MDC were positive to c-kit. Furthermore, in the area of MDC clusters, there were cells in the layer strongly positive for c-kit immunostaining (Figure 3B), implicating the source of MDC.

To further confirm the expression of stem cell markers in MDC, we performed RT-PCR to test the expression of different transcripts. c-kit was expressed in heart tissue, bone marrow cells, and MDCs. In addition, the other cardiac stem cell transcript sca-1 was undetectable in MDC; endothelial precursor marker gene CD34 was present in MDC. Cardiac transcripts α-MHC, Nkx2.5, and GATA4 were all detected in MDC, heart tissue and purified myocytes as well (Fig 3C; Fig S5).

### EXAMPLE 3-- Myocyte-derived cells re-differentiate

MDC self-organized into spheres 3-5 days after the cluster cells became more confluent. There were 0~4 spheres in each well of a 6-well culture plate, depending on the condition of cells. MDC spheres either loosely adhered to the culture layer or became suspended in medium, and show slow spontaneous activity within 2-5 days of sphere stage (Fig S4C. The semi-adherent spheres could be harvested by gentle pipetting. Semi-adherent or suspending spheres flattened onto the bottom when seeded into fibronectin-coated plates, and gave rise to cells off the spheres, which eventually stopped beating while turning into monolayer cells (Figure 4A). Moreover, myocyte cultures could provide 3~4 harvests of MDC or spheres. New daughter cells emerged again always around the area where previous MDC were produced.

In the spheres, most cells were positive for α-MHC, connexin 43 (Cx43), and CD31 immunostaining, and some positive for c-kit. Some cells off the sphere also express cTnT and others express c-kit (Fig 4B). When transduced with replication-defective lentivirus encoding enhanced green fluorescent protein (eGFP) driven by the cardiac α-MHC promoter, MDC spheres exhibited focal green florescence within 3-5 days along with spontaneous contraction (Fig 4D). RT-PCR revealed that in the spheres, there was weaker stem cell transcript signal of c-kit, but stronger signal of cardiac transcripts α-MHC, Nkx2.5, and GATA4, suggesting the cardiogenesis and re-differentiation of MDC when entering in sphere phase. In addition, endothelial precursor marker gene CD34, present in MDC, tended to decrease in the spheres; endothelial marker CD31 (PECAM-1) expresses in both MDCs and the spheres (Figure S5).

### EXAMPLE 4--EXPERIMENTAL PROCEDURES

### Isolation, purification, and primary culture of cardiomyocytes

Cardiomyocytes were isolated from adult male Wistar-Kyoto rats (4-8 weeks, 70-120 g), Hartley guinea pigs (3-5 weeks, 300-380 g) or C57BL/6 mice (4-6 weeks, 17-21 g) by enzymatic digestion of the whole heart on a Langendorff apparatus with similar protocol as previously described.(Zhang et al., 2006; Kizana et al., 2007) Heparinized animals were anaesthetized by sodium pentobarbital (Ovation Pharmaceuticals Inc, Deerfield, IL). Hearts were rapidly excised and cleaned to remove blood in ice-cold Tyrode's solution before mounted to a Langendorff apparatus conjugating to a pressure monitoring device, and perfused retrogradely with the following four oxygenated solutions in sequential order: modified Tyrode's solution containing 1.0 mM Ca₂⁺ (2 min), Ca₂⁺-free Tyrode's solution (2-3 min), Ca₂⁺-free Tyrode's solution containing 0.2 Wünsch unit/ml of collagenase made from Liberase Blendzyme 4 (Roche Molecular Biochemicals, Indianapolis, IN) for 10-20 min depending on species and digest conditions. Digested atrium and ventricles were cut off and minced in Kruftbrühe (KB) solution, then filtered through a 200 um nylon mesh to remove big piece of undigested tissues. Isolated cells were rinsed in KB solution and let settled by gravity for 3 times to remove debris and non-cardiomyocytes. Resuspended cells in KB solution were loaded above the top layer of Percoll gradient which was formed by 20%, 40%, and 70% of Percoll to separate myocytes from debris and other types of cells. After three washes in KB solution, myocytes were resuspended in KB solution or in culture media for further experiments. Modified Tyrode's solution contained (mM): NaCl 105, KCl 5.4, KH2PO4 0.6, NaH2PO4 0.6, NaHCO3 6, KHCO3 5, CaCl2 1, MgCl2 1, HEPES 10, glucose 5, taurine 20 (pH 7.35 with NaOH), and KB solution had (mM): KCl 20, KH2PO4 10, K-glutamate 70, MgC12 1, glucose 25, β-hydroxybutyric acid 10, taurine 20, EGTA 0.5, HEPES 10, and 0.1% albumin (pH 7.25 with KOH).

Purified myocytes were resuspended in Medium 199 (Invitrogen, Carlsbad, CA) supplemented with 110 mg/L sodium pyruvate, 0.1mM β-mercaptocthanol, 100 U/ml penicillin, 100 µg/ml streptomycin, and 5% FBS (Invitrogen) and cultured in laminin-coated 6-well culture plates or 100 mm dishes in normal density of 6000 and 9000 cells/cm² for ventricular and atrial myocytes respectively, at 37 °C for 1 hr before wash to remove dead and non-adherent cells, and repeated once after 1hr of culture. Serum concentration in medium was gradually increased to 10% and 20%. On the second and third day of plating, medium was replaced to remove dead cells, and then maintained for prolonged culture while partially changed about every 5 days.

### Cell imaging and tracking

In order to verify the proliferation of dedifferentiated myocytes, cells were plated in lower density as compared to normal dense culture for MDC production. Numeric grid-marked coverslips (Bellco Biotechnology, Vineland, NJ) coated with laminin were used to identify the cellular changes during the culture, under time-lapse microscope (Nikon TE-2000E inverted microscope) for continuous analysis, or under regular inverted microscope (Nikon TE-2000U), with phase contrast objectives and images were captured with a monochrome CCD camera (Q-Imaging, Surrey, BC, Canada) with a program suite Image Pro Plus (Media Cybernetics, Bethesda, MD). At the end of the tracking, cells were subjected to analysis of markers related to cell cycle progression and stem cell when needed. A 3CCD Color video camera (Sony) connected to a personal computer was used to capture real-time images and videos of beating cells and spheres.

### Culture of myocyte-derived cells

At about 10 days to 2 weeks after the culture, the loosely adherent myocytes-derived cells (MDC) were harvested by gentle pipetting for 3 times with a disposable transfer pipette. Cells were cultivated in same medium as of the serum-rich myocyte culture medium, for the experiments detecting the markers in fresh isolated cells. Alternatively, MDC culture medium which was DMEM/F12 supplemented with 0.1mM β-mercaptoethanol, bFGF 0.1 ng/ml, TGF- β 1 ng/ml, 100 U/ml penicillin, 100 µg/ml streptomycin, and 10% FBS, was used to maintain the cells in 95% humidity, 5% CO2, at 37C°.

### Labelling of myocytes with BrdU

Cells were loaded with 3-bromo-2-deoxyuridine (BrdU; 5 µM) for various periods before immunocytochemical assay (Engel et al., 2005).

### Fluorescent Immunocytochemistry

Cellular phenotypes in the cultures were analyzed similarly as previously described (Smith et al., 2007; Zhang et al., 2006) using immunofluorescence. To test the expression of stem cell markers, rabbit polyclonal antibody (pAb) against c-kit (CD117) (Santa Cruz Biotechnology, Santa Cruz, CA) or Oct-4 (Abcam, Cambridge, MA), mouse monoclonal antibody (mAb) against Sca-1 (Invitrogen), goat pAb against Thy-1 (CD90) were used as primary antibodies. Expression of cardiac markers were tested using antibodies included mouse mAb of cardiac specific α-MHC from Abcam, α-actin from Sigma, and rabbit pAb Cx43 and GATA4 from Invitrogen, goat pAb Nkx2.5 from Santa Cruz Biotechnologie, Inc. Primary antibodies against cell cycle-specific molecules included: Ki67, Histone H3 (phosphor S10) and anti-bromodeoxyuridine (BrdU) were from Abcam. The specificity of antibodies was confirmed by blocking peptides or control cells. Donkey anti-mouse, anti-rabbit, or anti-goat antibodies with fluorescent conjugation were used as secondary antibodies.

Direct immunostaining were also performed to test the expression of stem cell markers in freshly harvested MDC using PE-conjugated mouse mAbs against c-kit (BD Biosciences), Sca-1 (Invitrogen), or FITC-conjugated CD90 (Abcam).

In MDC spheres, stem cell and cardiac markers were detected using whole-mount immunofluorescent techniques and examined with standard and Z-stack confocal laser scan microscope (LSM 510; Zeiss). The acquisition settings were optimized to avoid false positive or false negative staining. Images were processed by LSM 510 software suite.

### RT-PCR

Reverse-transcription Polymerase Chain Reaction (RT-PCR) was performed to test the mRNA expression of both stem cell and cardiac markers. Extraction of total RNA from rat heart tissue, bone marrow cells flushed from femurs, purified myocytes, MDC, and MDC spheres, and one-step RT-PCR were carried out with commercially available kits (Qiagen, Valencia, CA). Primer pairs for c-kit, sca-1, Oct 4, α-MHC, GATA4, and NKx2.5, β-actin are listed in Table S1.

**Table S1. Primers used for RT-PCR detection**

| **Molecule** | **Access #** | **Primer** | | **Prod** | | |
|---|---|---|---|---|---|---|
| | | sense oligo 5' ....3' | antisense oligo 5'.....3' | start | stop | length |
| c-Kit * | NM022264 | (SEQ ID NO: 1) | GCGGACCAGTGCGTCGTTGTCTT (SEQ ID NO: 10) | 142 | 449 | 308 |
| sca-1 * | XM_343263. | CATCTTTCTCCTGGCCCTACT (SEQ ID NO: 2) | GAGGACTGAGCCCAGGATGAA (SEQ ID NO: 11) | 46 | 390 | 345 |
| CD90/Thy1 | NM_012673. | CCTGCCTGGTGAACCAGAACCTT (SEQ ID NO: 3) | GCAGGCTTATGCCACCACACTTG (SEQ ID NO: 12) | 125 | 451 | 327 |
| CD31 | NM_031591 | AGAAGGAAGAGACGGTGTTG (SEQ ID NO: 4) | TTAGGAGGCGGTAAGTGATG (SEQ ID NO: 13) | 1241 | 1498 | 258 |
| CD34 | XM_001070343 | TCAGAGACCACGGTCAACTT (SEQ ID NO: 5) | ACTCCTCGGATTCCTGAACA (SEQ ID NO: 14) | 417 | 721 | 305 |
| GATA4 | NM_144730 | TCTAAGACACCAGCAGGTCCTC (SEQ ID NO: 6) | TTGGAGCTGGCCTGTGAT (SEQ ID NO: 15) | 1540 | 1823 | 284 |
| NKx2.5 | NM_053651 | TTATCCGCGAGCCTACGGTGA (SEQ ID NO: 7) | CTGCCGCTGTCGCTTACACTT (SEQ ID NO: 16) | 366 | 684 | 319 |
| aMHC | NM_017239. | AGTCAGAGAAGGAGCGCCTA (SEQ ID NO: 8) | TAGATCATCCAGGCCGCATA (SEQ ID NO: 17) | 87 | 378 | 292 |
| b-actin* | NM_031144.2\| | ATATCGCTGCGCTCGTCGTC (SEQ ID NO: 9) | CGTCCCAGTTGGTGACAATG (SEQ ID NO: 18) | 92 | 322 | 231 |

### Statistics

Data were expressed as mean ± SEM, and paired or un-paired Student t-test were used to exam the significance of difference between groups, with a p<0.01 considered as significant different.

### References

The disclosure of each reference cited is expressly incorporated herein.
Ahuja,P., Sdek,P., and MacLellan,W.R. (2007). Cardiac myocyte cell cycle control in development, disease, and regeneration. Physiol Rev. 87, 521-544.
Ausma,J., Litjens,N., Lenders,M.H., Duimel,H., Mast,F., Wouters,L., Ramaekers,F., Allessie,M., and Borgers,M. (2001). Time course of atrial fibrillation-induced cellular structural remodeling in atria of the goat. J. Mol. Cell Cardiol. 33, 2083-2094.
Barile,L., Chimenti,I., Gaetani,R., Forte,E., Miraldi,F., Frati,G., Messina,E., and Giacomello,A. (2007). Cardiac stem cells: isolation, expansion and experimental use for myocardial regeneration. Nat. Clin. Pract. Cardiovasc. Med. 4 Suppl 1, S9-S14.
Beltrami,A.P., Urbanek,K., Kajstura,J., Yan,S.M., Finato,N., Bussani,R., Nadal-Ginard,B., Silvestri,F., Leri,A., Beltrami,C.A., and Anversa,P. (2001). Evidence that human cardiac myocytes divide after myocardial infarction. N. Engl. J. Med. 344, 1750-1757.
Benardeau,A., Hatem,S.N., Rucker-Martin,C., Tessier,S., Dinanian,S., Samuel,J.L., Coraboeuf,E., and Mercadier,J.J. (1997a). Primary culture of human atrial myocytes is associated with the appearance of structural and functional characteristics of immature myocardium. J. Mol. Cell Cardiol. 29, 1307-1320.
Benardeau,A., Hatem,S.N., Rucker-Martin,C., Tessier,S., Dinanian,S., Samuel,J.L., Coraboeuf,E., and Mercadier,J.J. (1997b). Primary culture of human atrial myocytes is associated with the appearance of structural and functional characteristics of immature myocardium. J. Mol. Cell Cardiol. 29, 1307-1320.
Bird,S.D., Doevendans,P.A., van Rooijen,M.A., Brutel,d.l.R., Hassink,R.J., Passier,R., and Mummery,C.L. (2003). The human adult cardiomyocyte phenotype. Cardiovasc. Res. 58, 423-434.
Boyle,A.J., Schulman,S.P., Hare,J.M., and Oettgen,P. (2006). Is stem cell therapy ready for patients? Stem Cell Therapy for Cardiac Repair. Ready for the Next Step. Circulation 114, 339-352.
Burton,P.B., Yacoub,M.H., and Barton,P.J. (1999). Cyclin-dependent kinase inhibitor expression in human heart failure. A comparison with fetal development. Eur. Heart J. 20, 604-611.
Chabner BA (1982). Cytosine arabinoside. In Pharmacologic Principles of Cancer Treatment, Chabner BA, ed. (Philadelphia, WB: Saunders Co.), pp. 387-401.
Darling,D.L., Yingling,J., and Wynshaw-Boris,A. (2005). Role of 14-3-3 proteins in eukaryotic signaling and development. Curr. Top. Dev. Biol. 68, 281-315.
de la Fuente,R., Abad,J.L., Garcia-Castro,J., Fernandez-Miguel,G., Petriz,J., Rubio,D., Vicario-Abejon,C., Guillen,P., Gonzalez,M.A., and Bernad,A. (2004). Dedifferentiated adult articular chondrocytes: a population of human multipotent primitive cells. Exp. Cell Res. 297, 313-328.
Dispersyn,G.D., Geuens,E., Ver,D.L., Ramaekers,F.C., and Borgers,M. (2001). Adult rabbit cardiomyocytes undergo hibernation-like dedifferentiation when co-cultured with cardiac fibroblasts. Cardiovasc. Res. 51, 230-240.
Dispersyn,G.D., Mesotten,L., Meuris,B., Maes,A., Mortelmans,L., Flameng,W., Ramaekers,F., and Borgers,M. (2002). Dissociation of cardiomyocyte apoptosis and dedifferentiation in infarct border zones. Eur. Heart J. 23, 849-857.
Driesen,R.B., Dispersyn,G.D., Verheyen,F.K., van den Eijnde,S.M., Hofstra,L., Thone,F., Dijkstra,P., Debie,W., Borgers,M., and Ramaekers,F.C. (2005). Partial cell fusion: a newly recognized type of communication between dedifferentiating cardiomyocytes and fibroblasts. Cardiovasc. Res. 68, 37-46.
Driesen,R.B., Verheyen,F.K., Dijkstra,P., Thone,F., Cleutjens,J.P., Lenders,M.H., Ramaekers,F.C., and Borgers,M. (2007). Structural remodelling of cardiomyocytes in the border zone of infarcted rabbit heart. Mol. Cell Biochem.
Driesen,R.B., Verheyen,F.K., Dispersyn,G.D., Thone,F., Lenders,M.H., Ramaekers,F.C., and Borgers,M. (2006). Structural adaptation in adult rabbit ventricular myocytes: influence of dynamic physical interaction with fibroblasts. Cell Biochem. Biophys. 44, 119-128.
Du,J., Liao,W., Wang,Y., Han,C., and Zhang,Y. (2005). Inhibitory effect of 14-3-3 proteins on serum-induced proliferation of cardiac fibroblasts. Eur. J. Cell Biol. 84, 843-852.
Engel,F.B., Schebesta,M., Duong,M.T., Lu,G., Ren,S., Madwed,J.B., Jiang,H., Wang,Y., and Keating,M.T. (2005). p38 MAP kinase inhibition enables proliferation of adult mammalian cardiomyocytes. Genes Dev. 19, 1175-1187.
Engel,F.B., Schebesta,M., and Keating,M.T. (2006). Anillin localization defect in cardiomyocyte binucleation. J. Mol. Cell Cardiol. 41, 601-612.
Fredj,S., Bescond,J., Louault,C., and Potreau,D. (2005). Interactions between cardiac cells enhance cardiomyocyte hypertrophy and increase fibroblast proliferation. J. Cell Physiol 202, 891-899.
Gartel,A.L., Serfas,M.S., and Tyner,A.L. (1996). p21--negative regulator of the cell cycle. Proc. Soc. Exp. Biol. Med. 213, 138-149.
Gruh,I., Beilner,J., Blomer,U., Schmiedl,A., Schmidt-Richter,I., Kruse,M.L., Haverich,A., and Martin,U. (2006). No evidence of transdifferentiation of human endothelial progenitor cells into cardiomyocytes after coculture with neonatal rat cardiomyocytes. Circulation 113, 1326-1334.
He,M., Zhang,J., Shao,L., Huang,Q., Chen,J., Chen,H., Chen,X., Liu,D., and Luo,Z. (2006). Upregulation of 14-3-3 isoforms in acute rat myocardial injuries induced by burn and lipopolysaccharide. Clin. Exp. Pharmacol. Physiol 33, 374-380.
Hermeking,H. and Benzinger,A. (2006). 14-3-3 proteins in cell cycle regulation. Semin. Cancer Biol. 16, 183-192.
Horackova,M. and Byczko,Z. (1997). Differences in the structural characteristics of adult guinea pig and rat cardiomyocytes during their adaptation and maintenance in long-term cultures: confocal microscopy study. Exp. Cell Res. 237, 158-175.
Kajstura,J., Leri,A., Finato,N., Di Loreto,C., Beltrami,C.A., and Anversa,P. (1998). Myocyte proliferation in end-stage cardiac failure in humans. Proc. Natl. Acad. Sci. U. S. A 95, 8801-8805.
Kang,S.K., Park,J.B., and Cha,S.H. (2006). Multipotent, dedifferentiated cancer stem-like cells from brain gliomas. Stem Cells Dev. 15, 423-435.
Kizana,E., Chang,C.Y., Cingolani,E., Ramirez-Correa,G.A., Sekar,R.B., Abraham,M.R., Ginn,S.L., Tung,L., Alexander,I.E., and Marban,E. (2007). Gene Transfer of Connexin43 Mutants Attenuates Coupling in Cardiomyocytes. Novel Basis for Modulation of Cardiac Conduction by Gene Therapy. Circ. Res.
Laframboise,W.A., Scalise,D., Stoodley,P., Graner,S.R., Guthrie,R.D., Magovern,J.A., and Becich,M.J. (2007). Cardiac fibroblasts influence cardiomyocyte phenotype in vitro. Am. J. Physiol Cell Physiol 292, C1799-C1808.
Laronga,C., Yang,H.Y., Neal,C., and Lee,M.H. (2000). Association of the cyclin-dependent kinases and 14-3-3 sigma negatively regulates cell cycle progression. J. Biol. Chem. 275, 23106-23112.
Lepilina,A., Coon,A.N., Kikuchi,K., Holdway,J.E., Roberts,R.W., Burns,C.G., and Poss,K.D. (2006). A dynamic epicardial injury response supports progenitor cell activity during zebrafish heart regeneration. Cell 127, 607-619.
Li,J.M. and Brooks,G. (1999). Cell cycle regulatory molecules (cyclins, cyclin-dependent kinases and cyclin-dependent kinase inhibitors) and the cardiovascular system; potential targets for therapy? Eur. Heart J. 20, 406-420.
Lien,C.L., Schebesta,M., Makino,S., Weber,G.J., and Keating,M.T. (2006). Gene expression analysis of zebrafish heart regeneration. PLoS. Biol. 4, e260.
Lyngbaek,S., Schneider,M., Hansen,J.L., and Sheikh,S.P. (2007). Cardiac regeneration by resident stem and progenitor cells in the adult heart. Basic Res. Cardiol. 102, 101-114.
Macleod,K.F., Sherry,N., Hannon,G., Beach,D., Tokino,T., Kinzler,K., Vogelstein,B., and Jacks,T. (1995). p53-dependent and independent expression of p21 during cell growth, differentiation, and DNA damage. Genes Dev. 9, 935-944.
Michalopoulos,G.K. and DeFrances,M.C. (1997). Liver regeneration. Science 276, 60-66.
Montessuit,C., Rosenblatt-Velin,N., Papageorgiou,I., Campos,L., Pellieux,C., Palma,T., and Lerch,R. (2004). Regulation of glucose transporter expression in cardiac myocytes: p38 MAPK is a strong inducer of GLUT4. Cardiovasc. Res. 64, 94-104.
Oh,H., Chi,X., Bradfute,S.B., Mishina,Y., Pocius,J., Michael,L.H., Behringer,R.R., Schwartz,R.J., Entman,M.L., and Schneider,M.D. (2004). Cardiac muscle plasticity in adult and embryo by heart-derived progenitor cells. Ann. N. Y. Acad. Sci. 1015, 182-189.
Poss,K.D. (2007). Getting to the heart of regeneration in zebrafish. Semin. Cell Dev. Biol. 18, 36-45.
Real,C., Glavieux-Pardanaud,C., Le Douarin,N.M., and Dupin,E. (2006). Clonally cultured differentiated pigment cells can dedifferentiate and generate multipotent progenitors with self-renewing potential. Dev. Biol.
Roninson,I.B. (2002). Oncogenic functions of tumour suppressor p21(Waf1/Cip1/Sdi1): association with cell senescence and tumour-promoting activities of stromal fibroblasts. Cancer Lett. 179, 1-14.
Rucker-Martin,C., Pecker,F., Godreau,D., and Hatem,S.N. (2002). Dedifferentiation of atrial myocytes during atrial fibrillation: role of fibroblast proliferation in vitro. Cardiovasc. Res. 55, 38-52.
Shyu,W.C., Lee,Y.J., Liu,D.D., Lin,S.Z., and Li,H. (2006). Homing genes, cell therapy and stroke. Front Biosci. 11, 899-907.
Smith,R.R., Barile,L., Cho,H.C., Leppo,M.K., Hare,J.M., Messina,E., Giacomello,A., Abraham,M.R., and Marban,E. (2007). Regenerative potential of cardiosphere-derived cells expanded from percutaneous endomyocardial biopsy specimens. Circulation 115, 896-908.
Straube,W.L. and Tanaka,E.M. (2006a). Reversibility of the differentiated state: regeneration in amphibians. Artif. Organs 30, 743-755.
Straube,W.L. and Tanaka,E.M. (2006b). Reversibility of the differentiated state: regeneration in amphibians. Artif. Organs 30, 743-755.
Thijssen,V.L., Ausma,J., and Borgers,M. (2001). Structural remodelling during chronic atrial fibrillation: act of programmed cell survival. Cardiovasc. Res. 52, 14-24.
Torella,D., Ellison,G.M., Mendez-Ferrer,S., Ibanez,B., and Nadal-Ginard,B. (2006). Resident human cardiac stem cells: role in cardiac cellular homeostasis and potential for myocardial regeneration. Nat. Clin. Pract. Cardiovasc. Med. 3 Suppl 1, S8-13.
Tseng,A.S., Engel,F.B., and Keating,M.T. (2006). The GSK-3 inhibitor BIO promotes proliferation in mammalian cardiomyocytes. Chem. Biol. 13, 957-963.
Von Harsdorf,R. (2001). Can cardiomyocytes divide? Heart 86, 481-482.
Walder,S., Zhang,F., and Ferretti,P. (2003). Up-regulation of neural stem cell markers suggests the occurrence of dedifferentiation in regenerating spinal cord. Dev. Genes Evol. 213, 625-630.
Welikson,R.E., Kaestner,S., Reinecke,H., and Hauschka,S.D. (2006). Human umbilical vein endothelial cells fuse with cardiomyocytes but do not activate cardiac gene expression. J. Mol. Cell Cardiol. 40, 520-528.
Yang,H., Zhang,Y., Zhao,R., Wen,Y.Y., Fournier,K., Wu,H.B., Yang,H.Y., Diaz,J., Laronga,C., and Lee,M.H. (2006). Negative cell cycle regulator 14-3-3sigma stabilizes p27 Kip1 by inhibiting the activity of PKB/Akt. Oncogene 25, 4585-4594.
Yeh,E.T., Zhang,S., Wu,H.D., Korbling,M., Willerson,J.T., and Estrov,Z. (2003). Transdifferentiation of human peripheral blood CD34+-enriched cell population into cardiomyocytes, endothelial cells, and smooth muscle cells in vivo. Circulation 108, 2070-2073.
Yoshizumi,M., Lee,W.S., Hsieh,C.M., Tsai,J.C., Li,J., Perrella,M.A., Patterson,C., Endege,W.O., Schlegel,R., and Lee,M.E. (1995). Disappearance of cyclin A correlates with permanent withdrawal of cardiomyocytes from the cell cycle in human and rat hearts. J. Clin. Invest 95, 2275-2280.
Zhang,Y., Han,H., Wang,J., Wang,H., Yang,B., and Wang,Z. (2003). Impairment of HERG (human ether-a-go-go related gene) K+ channel function by hypoglycemia and hyperglycemia: Similar phenotypes but different mechanisms. J. Biol. Chem. 278, 10417-10426.
Zhang,Y., Xiao,J., Wang,H., Luo,X., Wang,J., Villeneuve,L.R., Zhang,H., Bai,Y., Yang,B., and Wang,Z. (2006). Restoring depressed HERG K+ channel function as a mechanism for insulin treatment of abnormal QT prolongation and associated arrhythmias in diabetic rabbits. Am. J. Physiol Heart Circ. Physiol 291, H1446-H1455.
Zhao,J., Morozova,N., Williams,L., Libs,L., Avivi,Y., and Grafi,G. (2001). Two phases of chromatin decondensation during dedifferentiation of plant cells: distinction between competence for cell fate switch and a commitment for S phase. J. Biol. Chem. 276, 22772-22778.

### CLAUSES:

1. A method for obtaining mammalian stem-cell-like myocyte-derived cells (MDCs) from atrial or ventricular heart tissue, comprising the steps of:
   isolating cells from atrial or ventricular heart tissue to form a cell suspension; and
   culturing the cells in a medium comprising a mitogen thereby forming a composition comprising MDCs.
2. The method of clause 1 further comprising:
   harvesting cells at a plurality of time points from the medium to form a plurality of samples of MDCs;
   assessing the proliferative capacity of one or more of the samples of MDCs;
   clonally proliferating one or more of the samples of MDCs;
   testing one or more of the samples of MDCs to confirm expression of one or more marker of stem cells selected from the group consisting of c-kit, sca-1, MCR1, CD34, CD33, alpha-MHC, NKx2.5, GATA4, and CD105.
3. The method of clause 1 further comprising the step of:
   purifying the cell suspension to increase the proportion of myocytes in the cell suspension forming a purified myocyte preparation prior to culturing the cells.
4. The method of clause 1 further comprising clonally proliferating MDCs from the composition.
5. The method of clause 1 wherein the tissue is atrial heart tissue.
6. The method of clause 1 wherein the tissue is ventricular heart tissue.
7. The method of clause 1 wherein the heart tissue is an endomyocardial biopsy specimen.
8. The method of clause 1 wherein the heart tissue is a surgical biopsy specimen.
9. The method of clause 1 wherein the step of isolating employs enzymes to dissociate the cells from the heart tissue.
10. The method of clause 1 wherein the step of isolating employs mechanical means to dissociate the cells from the heart tissue.
11. The method of clause 1 wherein the step of purifying employs differential centrifugation in culture medium.
12. The method of clause 1 wherein the step of purifying employs culturing on a solid surface to reduce proportion of fibroblasts in the cell suspension.
13. The method of clause 1 wherein the step of purifying employs Percoll gradient centrifugation.
14. The method of clause 1 wherein the step of purifying employs culturing on a solid surface and Percoll gradient centrifugation of cells which do not adhere to the solid surface.
15. The method of clause 1 wherein the step of purifying employs resuspension of the cells in a culture medium with at least 0.1 to 20 % fetal bovine serum.
16. The method of clause 1 wherein the mitogen is selected from the group consisting of : human serum, fetal bovine serum, VEGF, HGF, IGF, FGF, EGF, GCSF, GMCSF, MCSF, CSF-I, and PDGF.
17. The method of clause 1 wherein the medium comprises at least 10% fetal bovine serum.
18. The method of clause 1 wherein the culturing is from 1-60 days.
19. The method of clause 2 further comprising the step of: administering cells from at least one of the samples of MDCs to a patient with heart disease.
20. The method of clause 4 further comprising the step of: administering cells from at least the composition comprising MDCs to a patient with heart disease.
21. The method of clause 4 wherein the patient has a condition selected from the group consisting of: chronic heart failure, post myocardial infarction, right ventricular failure, pulmonary hypertension, ventricular dysfunction induced by a cytotoxic agent, ventricular dysfunction induced by an anti-neoplastic agent.
22. The method of clause 4 further comprising the step of: delivering cells from at least one of the samples of MDCs by intracoronary infusion via a catheter.
23. The method of clause 4 further comprising the step of: delivering cells from at least one of the samples of MDCs by intramyocardial injection via a catheter.
24. The method of clause 4 further comprising the step of: delivering cells from at least one of the samples of MDCs by intramyocardial injection during surgery.
25. An isolated preparation of mammalian cardiac stem-like cells, wherein said cells proliferate in culture, have a diameter of 10-30 um, and wherein said cells express a marker selected from the group consisting of c-kit, NKx2.5, and GATA4.
26. The preparation of clause 25 wherein the cells do not express sca-1.
27. The preparation of clause 25 wherein said cells express c-kit, CD34, NKx2.5, and GATA4.
28. The preparation of clause 25 wherein said cells express a marker selected from sca-1, isl-1, SSEA-1, ABCG2, and alpha MHC.
29. The preparation of clause 25 wherein said cells express isl-1, SSEA-1, ABCG2, and alpha MHC.
30. The preparation of clause 25 wherein the cells are derived from adult cardiac myocytes.
31. The preparation of clause 25 wherein the cells are derived from adult atrial cardiac myocytes.
32. The preparation of clause 25 wherein the cells are derived from adult ventricular cardiac myocytes.
33. The preparation of clause 25 further comprising a matrix substrate.
34. The preparation of clause 25 further comprising a polymerizable matrix.
35. A method of treating heart disease comprising the step of: administering a preparation of cells according to clause 25 to a patient with heart disease.
36. The method of clause 35 wherein the patient has a condition selected from the group consisting of: chronic heart failure, post myocardial infarction, right ventricular failure, pulmonary hypertension, ventricular dysfunction induced by a cytotoxic agent, ventricular dysfunction induced by an anti-neoplastic agent.
37. The method of clause 35 wherein the step of administering is performed by intracoronary infusion via a catheter.
38. The method of clause 35 wherein the step of administering the preparation is performed by intramyocardial injection via a catheter.
39. The method of clause 35 wherein the step of administering the preparation is performed by intramyocardial injection during surgery.
40. The method of clause 35 wherein the preparation further comprises a matrix substrate.
41. The method of clause 35 wherein the preparation further comprises a polymerizable substrate.

## Claims

1. A preparation of mammalian cardiac stem-cell-like cells derived from myocytes (MDCs) isolated from the atria or ventricles of a non-embryonic mammalian heart,
wherein said MDCs are **characterized by** having one or more of the following features associated with undifferentiated stem cells:
expression of stem cell marker CD-34, expression of stem cell marker c-kit,
expression of early cardiac transcription factor GATA4,
expression of early cardiac transcription factor NKx2.5,
reentry into the cell cycle and, as compared to said myocytes,
reduced expression of cell cycle inhibitors,
reduced inward rectifier potassium current, and
reduced resting membrane potential,
wherein said features associated with undifferentiated stem cells are generated in said MDCs by culturing said isolated myocytes in the presence of a mitogen.

2. The cell preparation of claim 1, wherein said MDCs are further **characterized by** expression of endothelial marker CD 31.

3. The cell preparation of any one of the preceding claims, wherein said MDCs express reduced levels of one or more of the stem cell markers sca-1 and CD 90 as compared to said myocytes.

4. The cell preparation of any one of the preceding claims, wherein said cell cycle inhibitors comprise 14-3-3n and p21.

5. The cell preparation of any one of the preceding claims, wherein said reentry into the cell cycle is accompanied by an increase in one or more of expression of Ki-67, increase in histone 3-phosphate positive cells, and increase in BrdU uptake.

6. The cell preparation of any one of the preceding claims, wherein said MDCs range in diameter from about 10 microns to about 30 microns.

7. The cell preparation of any one of the preceding claims, wherein said mitogen is present in an amount from about 0.1% to about 20% of the total volume of said culture medium.

8. The cell preparation of any one of the preceding claims, wherein said mitogen used for culturing comprises one or more growth factors.

9. The cell preparation of claim 8, wherein said growth factors selected from the group consisting of one or more of the following: VEGF, HGV, IGF, FGF, EGF, GCSF, GMCSF, MCSF, CSF-1, and PDGF.

10. The cell preparation of any one of the preceding claims, wherein said MDCs are further capable of re-differentiation.

11. The cell preparation of claim 10, wherein said re-differentiation produces MDC-derived spheres that are either adherent or semi-adherent to a culture surface.

12. The cell preparation of claim 11, wherein said MDC-derived spheres are **characterized by** reduced expression of one or more of c-kit and CD34 as compared to MDCs and increased expression of one or more of alpha-MHC, GATA4 and NKx2.5 as compared to MDCs.

13. The cell preparation of any one of the preceding claims, wherein said myocytes are isolated by:
digesting mammalian atrial or ventricular tissue thereby creating an isolated cell suspension;
removing debris and non-cardiomyoctes from said isolated cells;
purifying myocytes by density centrifugation; and
resuspending said purified myocytes in culture media.

14. The cell preparation of any one of the preceding claims, wherein said preparation is suitable for administration to a mammal with heart disease in order treat the heart disease.

15. The composition of claim 14, wherein the heart disease comprises one or more of treatment of chronic heart failure, post-myocardial infarction, right ventricular failure, pulmonary hypertension, cytotoxicity-induced ventricular dysfunction, and ventricular dysfunction induced by an anti-neoplastic agent.


SEQUENCE LISTING

<110> MARBAN, Eduardo

<120> DEDIFFERENTIATION OF ADULT MAMMALIAN
  CARDIOMYOCYTES INTO CARDIAC STEM CELLS

<130> 201107.00009

<150> 60/858006
<151> 2006-11-09

<160> 18

<170> FastSEQ for Windows Version 4.0

<210> 1
<211> 23
<212> DNA
<213> Homo sapiens

<400> 1
agccgtctcc accatccatc cag                                                23

<210> 2
<211> 21
<212> DNA
<213> Homo sapiens

<400> 2
catctttctc ctggccctac t                                                  21

<210> 3
<211> 23
<212> DNA
<213> Homo sapiens

<400> 3
cctgcctggt gaaccagaac ctt                                                23

<210> 4
<211> 20
<212> DNA
<213> Homo sapiens

<400> 4
agaaggaaga gacggtgttg                                                    20

<210> 5
<211> 20
<212> DNA
<213> Homo sapiens

<400> 5
tcagagacca cggtcaactt                                                    20

<210> 6
<211> 22
<212> DNA
<213> Homo sapiens

<400> 6
tctaagacac cagcaggtcc tc                                                 22

<210> 7
<211> 21
<212> DNA
<213> Homo sapiens

<400> 7
ttatccgcga gcctacggtg a                                                21

<210> 8
<211> 20
<212> DNA
<213> Homo sapiens

<400> 8
agtcagagaa ggagcgccta                                                  20

<210> 9
<211> 20
<212> DNA
<213> Homo sapiens

<400> 9
atatcgctgc gctcgtcgtc                                                  20

<210> 10
<211> 23
<212> DNA
<213> Homo sapiens

<400> 10
gcggaccagt gcgtcgttgt ctt                                              23

<210> 11
<211> 21
<212> DNA
<213> Homo sapiens

<400> 11
gaggactgag cccaggatga a                                                21

<210> 12
<211> 23
<212> DNA
<213> Homo sapiens

<400> 12
gcaggcttat gccaccacac ttg                                              23

<210> 13
<211> 20
<212> DNA
<213> Homo sapiens

<400> 13
ttaggaggcg gtaagtgatg                                                  20

<210> 14
<211> 20
<212> DNA
<213> Homo sapiens

<400> 14
actcctcgga ttcctgaaca                                                  20

<210> 15
<211> 18
<212> DNA
<213> Homo sapiens

<400> 15
ttggagctgg cctgtgat                                                    18

<210> 16
<211> 21
<212> DNA
<213> Homo sapiens

<400> 16
ctgccgctgt cgcttacact t                                                21

<210> 17
<211> 20
<212> DNA
<213> Homo sapiens

<400> 17
tagatcatcc aggccgcata                                                  20

<210> 18
<211> 20
<212> DNA
<213> Homo sapiens

<400> 18
cgtcccagtt ggtgacaatg                                                  20